# FASCICULE DE BREVET EUROPEEN

(11) **EP 2 513 049 B1**
(45) Date de publication et mention de la délivrance du brevet: **05.09.2018**
(21) Numéro de dépôt: 10787127.9
(22) Date de dépôt: 07.12.2010
(51) Int. Cl.: C07C 409/14, C07C 407/00

(54) **PROCÉDÉ D'OXYDATION D'HYDROCARBURES PAR L'OXYGÈNE**
VERFAHREN ZUR OXIDIERUNG VON KOHLENWASSERSTOFFEN MIT SAUERSTOFF
METHOD FOR OXIDIZING HYDROCARBONS WITH OXYGEN

(30) Priorité: 17.12.2009 FR 0959113
(43) Date de publication de la demande: 24.10.2012
(73) Titulaire: RHODIA OPERATIONS, 93306 Aubervilliers (FR)
(72) Inventeur: LE PORT, Philippe, 69960 Corbas (FR); MANTE, Thierry, 201702 Shanghai (CN)
(74) Mandataire: Chatelan, Florence Anne
(86) Numéro de dépôt international: PCT/EP2010/069031
(87) Numéro de publication internationale: WO 2011/073053

(56) Documents cités:
- EP-A1- 0 031 113
- US-A- 3 510 526

## Description

La présente invention concerne un procédé d'oxydation par l'oxygène d'hydrocarbures cycliques saturés tels que le cyclohexane pour la production d'hydroperoxyde d'alkyle.
Elle concerne plus particulièrement un procédé d'oxydation par l'oxygène d'hydrocarbures saturés, l'oxydation étant réalisée en plusieurs étapes successives pour contrôler l'avancement de la réaction d'oxydation de l'hydrocarbure et pour obtenir une bonne sélectivité en hydroperoxyde d'alkyle.
Ainsi, le procédé d'oxydation peut être réalisé dans plusieurs dispositifs tels qu'un dispositif comprenant plusieurs réacteurs montés en série ou en cascade ou une colonne à bulles compartimentée comprenant plusieurs étages définis par des plateaux, ou analogues. Une telle mise en oeuvre du procédé d'oxydation permet d'obtenir une sélectivité élevée en hydroperoxyde d'alkyle.
Cette étape d'oxydation forme souvent la première étape d'un procédé permettant de fabriquer des composés oxydés tels que des cétones, alcools, aldéhydes et acides. Ainsi, l'oxydation du cyclohexane par l'oxygène pour former l'hydroperoxyde de cyclohexyle est la première étape du procédé de fabrication du cyclohexanol et de la cyclohexanone, qui peuvent eux-mêmes être des intermédiaires de synthèse, par exemple de l'acide adipique ou de l'epsilon-caprolactame.
Dans les procédés de fabrication de l'acide adipique, un des plus utilisés à l'échelle industrielle consiste à oxyder par l'oxygène moléculaire, le cyclohexane en hydroperoxyde de cyclohexyle en présence ou non de catalyseur, puis à décomposer catalytiquement l'hydroperoxyde en un mélange de cyclohexanone et cyclohexanol. Le mélange de cétone et d'alcool est ensuite oxydé en acide adipique par l'acide nitrique.
La première étape d'oxydation du cyclohexane est généralement réalisée en milieu biphasique gaz/liquide, le gaz oxydant, de l'oxygène ou un gaz contenant de l'oxygène, est introduit dans le milieu liquide dans des réacteurs constitués d'une ou plusieurs colonnes à bulles compartimentées ou non, montées en série lorsque leur nombre est supérieur à un, fonctionnant soit à co-courant, soit à contre-courant du sens de circulation de la phase liquide constituée principalement par du cyclohexane à l'état liquide. Cette étape est notamment décrite dans les brevets GB 777087, 1112837, 964869, 1191573, US 3479394, US 4877903.
Le milieu réactionnel liquide contenant les produits oxydés dont principalement l'hydroperoxyde d'alkyle est récupéré généralement en tête de chaque réacteur.
Chaque réacteur contient également au moins une phase gazeuse formant le "ciel" de la zone de réaction.

Pour obtenir un rendement et une sélectivité convenables en hydroperoxyde d'alkyle, il est favorable de réaliser la réaction avec un faible taux de conversion de l'hydrocarbure dans chaque réacteur ou compartiment pour avoir dans le milieu réactionnel une concentration basse en produits oxydés. Pour ce faire, il faut maîtriser le dégagement de chaleur induit par la réaction.

En effet, une concentration élevée en produits oxydés accélère la vitesse de la réaction et dégrade sa sélectivité en produits valorisables tels que : hydroperoxyde d'alkyle, alcool, cétone. L'élévation non maîtrisée de température accélère également la vitesse de la réaction et favorise la déperoxydation de l'hydroperoxyde d'alkyle.

De ce fait, pour obtenir un taux de production d'hydroperoxyde d'alkyle compatible avec une exploitation industrielle, la réaction d'oxydation est généralement réalisée dans plusieurs réacteurs montés en série ou en cascade ou dans une colonne à bulles compartimentée par la présence de plateaux, ou analogues.

Dans un tel procédé, il est important de contrôler d'une part, la température dans chaque réacteur ou chaque compartiment pour maintenir le milieu réactionnel dans le domaine optimal et d'autre part, la concentration en produits oxydés dans le milieu réactionnel. Cette régulation peut être, par exemple, obtenue par alimentation d'hydrocarbure à une température plus basse dans un ou plusieurs réacteurs ou compartiments, notamment les derniers réacteurs de la cascade ou derniers compartiments de la colonne.

Pour l'économie du procédé, il est également important de récupérer la phase gazeuse formant le ciel des réacteurs ou compartiments et recycler les produits présents dans ces phases gazeuses tels que l'hydrocarbure non oxydé, les produits oxydés comme les hydroperoxydes, alcools, et cétones. Toutefois, le recyclage de ces produits récupérés ne doit pas perturber ou diminuer les performances du procédé et notamment les conditions de fonctionnement du réacteur ou compartiment dans lequel a lieu ledit recyclage.

Le brevet US 3510526 décrit un procédé de préparation d'hydroperoxyde de cyclohexyle à partir de cyclohexane dans un réacteur d'oxydation, le cyclohexane non oxydé étant mis en contact avec une solution aqueuse d'agent basique.

Un des buts de la présente invention est de proposer un procédé d'oxydation d'un hydrocarbure saturé mis en oeuvre dans un dispositif permettant de réaliser l'oxydation en plusieurs étapes successives tel que, par exemple, plusieurs réacteurs montés en série ou une ou plusieurs colonnes à bulles compartimentées et comprenant un processus de récupération et recyclage de phases gazeuses formant le ciel des réacteurs ou des compartiments de la colonne présentant de nombreux avantages pour l'économie globale du procédé.

A cet effet, l'invention propose un procédé d'oxydation par l'oxygène d'hydrocarbure saturé pour produire un hydroperoxyde d'alkyle. Ce procédé consiste à mettre en contact un hydrocarbure en phase liquide avec de l'oxygène ou un gaz contenant de l'oxygène, cette réaction étant réalisée en plusieurs étapes successives. Dans un tel procédé, l'hydrocarbure saturé est alimenté au moins dans la première étape à une température comprise entre 100 et 250°C, de préférence entre 150 et 200°C lorsque la réaction est mise en oeuvre en l'absence de catalyseur. En outre, au moins dans la dernière étape, le milieu réactionnel est refroidi pour maintenir la température du milieu réactionnel à un niveau inférieur ou égal à celui de la première étape pour obtenir une sélectivité convenable en hydroperoxyde d'alkyle. De préférence, au moins dans la dernière étape, le milieu réactionnel est refroidi pour maintenir la température du milieu réactionnel à un niveau inférieur à celui de la première étape.
Le procédé de l'invention se caractérise en ce que les phases gazeuses formant le ciel dans chaque étape de réaction sont récupérées et au moins partiellement condensées soit séparément soit après mélange de phases gazeuses issues de plusieurs étapes de réaction. Le ou les condensats obtenus sont recyclés dans au moins une étape dont la concentration en produits oxydés dans le milieu réactionnel alimenté dans ladite étape est au moins égale à la concentration en produits oxydés contenus dans le ou les condensats à recycler. Le ou les condensats obtenus sont recyclés directement dans au moins une étape dont la concentration en produits oxydés dans le milieu réactionnel alimenté dans ladite étape est au moins égale à la concentration en produits oxydés contenus dans le ou les condensats à recycler. Par directement, on entend au sens de la présente invention, que le ou les condensats ne subissent aucun traitement de purification intermédiaire, notamment de distillation.
Selon un mode de réalisation de l'invention, la phase gazeuse récupérée en tête d'une étape de réaction est condensée partiellement et le condensat obtenu est recyclé à l'alimentation de l'étape précédente.
Ainsi notamment, la phase gazeuse récupérée en tête de la dernière étape de réaction est condensée partiellement, le condensat obtenu est avantageusement recyclé dans une des étapes située en amont, notamment à l'alimentation de l'étape précédente.
Le recyclage de ce condensat permet notamment de contrôler et maintenir la température dans ladite étape de réaction dans le domaine optimal décrit précédemment et sans augmenter la concentration en produits oxydés dans le milieu réactionnel. En effet, la température de ces condensats est inférieure à celle du milieu réactionnel présent dans ladite étape et donc permet de maintenir la température dans un domaine diminuant la rapidité d'avancement de la réaction d'oxydation et donc améliorer la sélectivité en hydroperoxyde d'alkyle. En effet, cette sélectivité décroit très rapidement avec le degré d'avancement de la réaction. Le domaine de température optimal est déterminé pour avoir un effet autocatalytique de la réaction minimal.
Dans les procédés connus, ce contrôle de la température est généralement obtenu par alimentation d'hydrocarbure froid dans la dite étape. Les sources d'hydrocarbure froid disponibles sont constituées des distillats assurant, dans l'aval du procédé, le recyclage de l'hydrocarbure non transformé. Cet hydrocarbure froid est donc un produit purifié par distillation.

Avec le procédé de l'invention, la quantité nécessaire d'hydrocarbure recyclé et purifié à alimenter dans ladite étape est fortement diminuée, même dans certains cas réduite à zéro.

En outre, l'hydrocarbure froid recyclé et purifié alimenté permettait de maintenir la concentration en produits oxydés dans ladite étape à une valeur inférieure à une valeur critique pour la sélectivité et la vitesse de la réaction.

Cet effet est également obtenu avec le recyclage des condensats conformément à l'invention car ces condensats présentent une concentration en produits oxydés au plus égale à celle présente dans le milieu réactionnel alimenté à la dite étape et donc permettent de maîtriser la concentration en produits oxydés dans la dite étape.

En outre, les produits lourds présents dans les phases gazeuses sont condensés et donc recyclés uniquement dans les dernières étapes de la réaction. Ainsi, la réaction d'oxydation est réalisée dans les premières étapes avec une concentration faible en produits lourds. Cette faible concentration permet également d'améliorer la sélectivité globale de la réaction.

Le procédé de l'invention permet donc de diminuer la quantité d'hydrocarbure recyclé et purifié utilisée pour refroidir les milieux réactionnels dans certaines étapes de la réaction. Cet hydrocarbure purifié pourra donc être directement alimenté dans la première étape de la réaction d'oxydation, améliorant la sélectivité de la réaction et l'économie du procédé.

En outre, les phases gazeuses ou condensats recyclés dans les premières étapes du procédé contiennent peu de produits dit « lourds », ce qui permet également d'améliorer la sélectivité en hydroperoxyde de la réaction d'oxydation.

Comme la sélectivité globale du procédé est améliorée, la quantité d'hydrocarbure consommée par tonne de cyclohexanol et cyclohexanone produite est diminuée.

Dans un mode de réalisation de l'invention, il est avantageux de récupérer et mélanger les phases gazeuses formant le ciel de plusieurs étapes de la réaction d'oxydation et de les mélanger avant de les condenser partiellement. Le condensat récupéré est avantageusement recyclé dans une des étapes dont la concentration en produits oxydés dans le mélange réactionnel alimenté est supérieure ou égale à celle dans le condensat à recycler.

Toutefois, il est possible de réaliser toutes les combinaisons et associations possibles des phases gazeuses récupérées sans sortir du cadre de l'invention.
En outre, il est également possible de condenser chaque phase gazeuse séparément et de recycler les différents condensats obtenus de manière séparée ou après avoir les avoir mélangés ou réaliser des mélanges de certains d'entre eux.
Selon une autre caractéristique de l'invention, les hydrocarbures saturés convenables pour l'invention sont les hydrocarbures cycliques saturés tels que le cyclohexane, le cyclooctane, le cyclododécane, la décaline. De préférence, l'hydrocarbure saturé convenable pour l'invention est le cyclohexane.
Le cyclohexane est l'hydrocarbure utilisé pour la production d'hydroperoxyde de cyclohexyle, intermédiaire pour, notamment, la production de cyclohexanone et/ou cyclohexanol. Ce mélange d'alcool/cétone est par exemple utilisé pour la synthèse d'acide adipique, par oxydation nitrique.
Dans le cas de l'oxydation du cyclohexane mise en oeuvre sans catalyseur, les phases gazeuses récupérées au niveau des différentes étapes de la réaction sont avantageusement condensées partiellement à une température supérieure à la température de condensation de l'acide formique. En effet, cet acide, qui se forme au cours de l'étape d'oxydation, joue un rôle défavorable vis-à-vis de la sélectivité de la réaction en catalysant la déperoxydation de l'hydroperoxyde.
Selon une autre caractéristique de l'invention, les fractions gazeuses récupérées après condensation partielle des phases gazeuses recueillies en sortie des différentes étapes et recyclages des phases condensées ou condensats dans les différentes étapes d'oxydation, sont soumises à une deuxième condensation totale. Le condensat est ensuite flashé pour récupérer, en sus des incondensables, d'une part une phase organique qui est avantageusement recyclée à la première étape de l'oxydation, avantageusement après mélange avec l'hydrocarbure recyclé provenant des différentes distillations en aval de l'oxydation, d'autre part une phase aqueuse constituée de l'eau formée au cours de l'oxydation contenant la grande majorité de l'acide formique. Cette phase aqueuse est avantageusement détruite par exemple par incinération.
Par produits oxydés, on entend les composés obtenus par oxydation de l'hydrocarbure et comprenant des atomes d'oxygène. Ces produits sont notamment les hydroperoxydes d'alkyle, alcools, cétones, aldéhydes, acides et analogues.
Le procédé de l'invention permet de produire un hydroperoxyde d'alkyle, notamment l'hydroperoxyde de cyclohexyle avec un rendement convenable et une consommation de cyclohexane par tonne de cyclohexanol et cyclohexanone produits plus faible que celle constatée lorsque le recyclage des condensats issus des phases gazeuses formant le ciel des différentes étapes d'oxydation mis en oeuvre selon le procédé de l'invention est remplacé par un appoint de cyclohexane froid recyclé et purifié en aval de l'étape d'oxydation.

Le procédé de l'invention peut être mis en oeuvre en continu dans différents types d'installations comme décrit ultérieurement. Le nombre de réacteurs et le type de réacteur utilisé ne sont pas critiques pour l'invention.

Selon des caractéristiques préférées de l'invention, les types d'installation préférés sont une installation comprenant un réacteur d'oxydation constitué par une colonne à bulles compartimentée par des plateaux ou une installation comprenant plusieurs réacteurs d'oxydation montés en cascade ou série, chaque réacteur peut être du type colonne à bulles. Ainsi, chaque espace délimité par deux plateaux adjacents ou chaque réacteur correspond à une étape de la réaction d'oxydation. Le nombre de plateaux dans une colonne ou le nombre de réacteurs formant une cascade de réacteur est variable et peut être avantageusement compris entre 3 et 10.

D'autres détails, avantages de l'invention apparaitront plus clairement au vu des exemples donnés ci-dessous uniquement à titre d'illustration.

### Exemple comparatif n°1

Dans une installation d'oxydation de cyclohexane en hydroperoxyde de cyclohexyle comprenant une cascade de n réacteurs du type colonne à bulles avec alimentation à co-courant du gaz oxydant (air) et du milieu réactionnel à oxyder, un flux de cyclohexane chaud est alimenté dans le premier réacteur de la colonne à une température de 182°C. Le flux de cyclohexane chaud contient 0,31 % poids d'hydroperoxyde de cyclohexyle, cyclohexanol et cyclohexanone.

La réaction d'oxydation est mise en oeuvre sans catalyseur. Le milieu réactionnel liquide sortant d'un réacteur est alimenté dans le réacteur suivant de la cascade. Les phases gazeuses ou gaz d'oxydation de chaque réacteur sont récupérées et condensées, certaines après mélange.

La température dans le dernier réacteur n de la cascade est de 180°C. Pour maintenir la température à cette valeur, du cyclohexane « froid », à 70°C, est alimenté, en complément du milieu réactionnel, dans les deux derniers réacteurs n et n-1 de la cascade. Le flux de cyclohexane « froid » alimenté dans chaque réacteur représente 8,3 % en poids du flux de cyclohexane « chaud » alimenté dans le premier réacteur de la cascade. En sortie du dernier réacteur d'oxydation, une partie du cyclohexane n'ayant pas réagi est séparée du mélange réactionnel pour être recyclée à l'oxydation. Le mélange réactionnel est ensuite lavé à l'eau, puis traité avec un catalyseur pour transformer l'hydroperoxyde de cyclohexyle en cyclohexanol et cyclohexanone. Le cyclohexane est ensuite séparé par distillation, puis le mélange cyclohexanol - cyclohexanone brut est purifié par distillation.

Les gaz d'oxydation sont mélangés et condensés dans un train de condenseurs. La fraction liquide récupérée est flashée. La phase organique récupérée après séparation de l'eau est recyclée dans le cyclohexane alimenté dans le premier réacteur de la cascade avant l'étape de chauffage du cyclohexane.

### Exemple 1 conforme à l'invention :

L'exemple 1 est réalisé dans l'installation décrite à l'exemple comparatif avec les mêmes réactifs.

Toutefois, conformément à l'invention, les gaz d'oxydation ou phase gazeuse récupérés dans le dernier réacteur n de la cascade sont condensés séparément. Le condensat ainsi récupéré à une température de 145°C, représentant 4,5 % du débit de cyclohexane chaud alimenté au premier réacteur, est mélangé avec le milieu réactionnel alimenté au réacteur n-1 en complément et remplacement partiel du cyclohexane « froid » alimenté dans ce réacteur.

Les gaz d'oxydation ou phases gazeuses des autres réacteurs sont mélangés et condensés. La fraction la moins volatile de ces condensats, récupérée à une température de 125°C, représentant 11,5 % du débit de cyclohexane chaud alimenté au premier réacteur, est recyclée dans le milieu réactionnel entrant dans le réacteur n-2. Le reste des condensats est traité de manière similaire à celle décrite dans l'exemple comparatif ci-dessus. La phase organique finalement récupérée est mélangée au cyclohexane résultant des différents recyclages en aval de l'oxydation, avant chauffage de ce cyclohexane pour alimentation au premier réacteur de la cascade.

Avec le recyclage d'au moins une partie des condensats, tel que décrit ci-dessus, le cyclohexane « chaud » alimenté dans le premier réacteur contient 0,19% poids d'hydroperoxyde de cyclohexyle, cyclohexanol et cyclohexanone. La température dans le premier réacteur est égale à 185°C, la température du dernier réacteur restant maintenue à 180°C.

Les débits de cyclohexane « froid » alimenté dans les deux derniers réacteurs de la cascade représentent, respectivement, 6,5 et 5,5 % du débit de cyclohexane chaud alimenté au premier réacteur.

La sélectivité du procédé en cyclohexanol et cyclohexanone calculée après l'étape de transformation de l'hydroperoxyde de cyclohexyle est augmentée de 1%. La sélectivité représente le pourcentage de cyclohexane consommé et transformé en cyclohexanol et cyclohexanone. Ce gain de sélectivité en cyclohexanol et cyclohexanone est essentiellement obtenu par la diminution de la concentration en produits oxydés dans le cyclohexane chaud alimenté au premier réacteur de la cascade.

### Exemple comparatif n° 2

Dans une autre installation d'oxydation de cyclohexane en hydroperoxyde de cyclohexyle comprenant une cascade de n réacteurs du type colonne à bulles avec alimentation à co-courant du gaz oxydant (air) et du milieu réactionnel à oxyder, un flux de cyclohexane chaud est alimenté dans le premier réacteur de la colonne à une température de 185°C. Le flux de cyclohexane chaud contient 0,20 % poids d'hydroperoxyde de cyclohexyle, cyclohexanol et cyclohexanone.

La réaction d'oxydation est mise en oeuvre sans catalyseur. Le milieu réactionnel liquide sortant d'un réacteur est alimenté dans le réacteur suivant de la cascade. Les phases gazeuses ou gaz d'oxydation de chaque réacteur sont récupérées et condensées, certaines après mélange.

La température dans le dernier réacteur n de la cascade est de 179°C. Pour maintenir la température à cette valeur, du cyclohexane « froid », à 70°C, est alimenté, en complément du milieu réactionnel, dans les trois derniers réacteurs n-2 , n-1 , et n de la cascade. Le flux de cyclohexane « froid » alimenté dans chaque réacteur représente 2,7 , 3,1 , et 2,7 % en poids du flux de cyclohexane « chaud » alimenté dans le premier réacteur de la cascade, soit 8,5 % au total.

En sortie du dernier réacteur d'oxydation, une partie du cyclohexane n'ayant pas réagi est séparée du mélange réactionnel pour être recyclée à l'oxydation. Le mélange réactionnel est ensuite lavé à l'eau, puis traité avec un catalyseur pour transformer l'hydroperoxyde de cyclohexyle en cyclohexanol et cyclohexanone. Le cyclohexane est ensuite séparé par distillation, puis le mélange cyclohexanol - cyclohexanone brut est purifié par distillation.

Les gaz d'oxydation sont mélangés et condensés dans un train de condenseurs. La fraction liquide récupérée est flashée. La phase organique récupérée après séparation de l'eau est recyclée dans le cyclohexane alimenté dans le premier réacteur de la cascade avant l'étape de chauffage du cyclohexane.

### Exemple 2 conforme à l'invention :

L'exemple 2 est réalisé dans l'installation décrite à l'exemple comparatif n° 2 avec les mêmes réactifs.

Les gaz d'oxydation ou phases gazeuses des n-1 premiers réacteurs sont mélangés et condensés. La fraction la moins volatile de ces condensats, récupérée à une température de 125°C, représentant 8,1 % du débit de cyclohexane chaud alimenté au premier réacteur, est recyclée dans le milieu réactionnel entrant dans le réacteur n-2. Le reste des condensats est traité de manière similaire à celle décrite dans l'exemple comparatif ci-dessus. La phase organique finalement récupérée est mélangée au cyclohexane résultant des différents recyclages en aval de l'oxydation, avant chauffage de ce cyclohexane pour alimentation au premier réacteur de la cascade.

Avec le recyclage d'au moins une partie des condensats, tel que décrit ci-dessus, le cyclohexane « chaud » alimenté dans le premier réacteur contient 0,16 % poids d'hydroperoxyde de cyclohexyle, cyclohexanol et cyclohexanone. La température dans le premier réacteur est égale à 186,5°C, la température du dernier réacteur restant maintenue à 179°C.

Du cyclohexane « froid » est alimenté dans les réacteurs n-3, n-2, n-1 et n de la cascade avec des débits représentant respectivement 1 , 0,5 , 2,7 , et 1,6 % du débit de cyclohexane chaud alimenté au premier réacteur, soit 5,8 % au total.

La sélectivité du procédé en cyclohexanol et cyclohexanone calculée après l'étape de transformation de l'hydroperoxyde de cyclohexyle est augmentée de 0,6 %. La sélectivité représente le pourcentage de cyclohexane consommé et transformé en cyclohexanol et cyclohexanone. Ce gain de sélectivité en cyclohexanol et cyclohexanone est essentiellement obtenu par la diminution de la concentration en produits oxydés dans le cyclohexane chaud alimenté au premier réacteur de la cascade.

## Revendications

1. Procédé de fabrication d'hydroperoxyde d'alkyle par oxydation d'un hydrocarbure saturé en phase liquide par un gaz contenant de l'oxygène, la réaction étant mise en oeuvre dans plusieurs étapes successives, l'hydrocarbure étant alimenté au moins dans la première étape à une température comprise entre 100 et 250°C, au moins la dernière étape de la réaction étant refroidie pour maintenir et contrôler la température dans ladite étape à un niveau inférieur ou égal à celui de la première étape, **caractérisé en ce que** chaque étape de la réaction comprend une phase liquide et une phase gazeuse formant un ciel gazeux dans ladite étape, ladite phase gazeuse formant le ciel étant soutirée et évacuée pour être condensée au moins partiellement et **en ce que** le condensat récupéré à partir de la phase gazeuse d'une étape est recyclé dans une desdites étapes d'oxydation dont la concentration en produits oxydés dans le milieu réactionnel alimenté est au moins égal à la concentration en produits oxydés dans le condensat à recycler; le procédé étant **caractérisé en outre en ce que** le condensat est recyclé directement dans au moins une étape dont la concentration en produits oxydés dans le milieu réactionnel alimenté dans ladite étape est au moins égale à la concentration en produits oxydés contenus dans le condensat à recycler et **en ce que** l'hydrocarbure saturé est choisi dans le groupe comprenant le cyclohexane, le cyclooctane, le cyclododécane et la décaline.

2. Procédé selon la revendication 1, **caractérisé en ce que** le condensat recyclé dans une étape est obtenu par mélange de condensats ou de phases gazeuses provenant d'au moins deux étapes d'oxydation.

3. Procédé selon la revendication 1 ou 2, **caractérisé en ce que** la phase gazeuse récupérée en tête d'une étape de réaction est condensée partiellement et le condensat obtenu est recyclé à l'alimentation de l'étape précédente.

4. Procédé selon l'une des revendications 1 à 3, **caractérisé en ce que** la phase gazeuse récupérée en tête de la dernière étape de réaction est condensée partiellement et le condensat obtenu est recyclé à l'alimentation de l'étape précédente.

5. Procédé selon la revendication 1, **caractérisé en ce que** l'hydrocarbure saturé est le cyclohexane.

6. Procédé selon l'une des revendications précédentes, **caractérisé en ce qu'**après condensation partielle des phases gazeuses forment le ciel de chaque étape, la fraction non condensée est condensée totalement, puis ledit condensat obtenu est flashé pour obtenir une fraction liquide aqueuse et une fraction organique, ladite fraction organique étant recyclée dans la première étape d'oxydation.

7. Procédé selon l'une des revendications précédentes, **caractérisé en ce que** l'oxydation est réalisée dans une installation comprenant plusieurs réacteurs d'oxydation montés en série ou cascade, chaque réacteur formant une étape d'oxydation.

8. Procédé selon la revendication 7, **caractérisé en ce que** les réacteurs sont du type colonne à bulles.

9. Procédé selon l'une des revendications 1 à 6, **caractérisé en ce que** l'oxydation est réalisée dans une installation comprenant comme réacteur d'oxydation, une colonne à bulle compartimentée, chaque compartiment formant une étape d'oxydation.

## Patentansprüche

1. Verfahren zur Herstellung von Alkylhydroperoxyd durch Oxidation eines gesättigten Kohlenwasserstoffs in der flüssigen Phase durch ein sauerstoffhaltiges Gas, wobei die Reaktion in mehreren aufeinanderfolgenden Stufen durchgeführt wird, wobei der Kohlenwasserstoff wenigstens in der ersten Stufe mit einer Temperatur zwischen 100 und 250 °C zugeführt wird, wobei wenigstens die letzte Reaktionsstufe gekühlt wird, um die Temperatur in der Stufe auf einem Niveau unter oder gleich jenem der ersten Stufe zu halten und zu kontrollieren, **dadurch gekennzeichnet, dass** jede Reaktionsstufe eine flüssige Phase und eine gasförmige Phase umfasst, die in der Stufe eine gasförmige Atmosphäre bildet, wobei die die Atmosphäre bildende gasförmige Phase abgezogen und evakuiert wird, um wenigstens teilweise kondensiert zu werden, und dass das aus der gasförmigen Phase einer Stufe rückgewonnene Kondensat zu einer der Oxidationsstufen recycelt wird, deren Konzentration an Oxidationsprodukten in dem zugeführten Reaktionsmedium wenigstens gleich der Konzentration an Oxidationsprodukten in dem zu recycelnden Kondensat ist, wobei das Verfahren ferner **dadurch gekennzeichnet ist, dass** das Kondensat direkt zu wenigstens einer Stufe recycelt wird, deren Konzentration an Oxidationsprodukten in dem in der Stufe zugeführten Reaktionsmedium wenigstens gleich der Konzentration an Oxidationsprodukten ist, die in dem zu recycelnden Kondensat enthalten sind, und dass der gesättigte Kohlenwasserstoff aus der Gruppe ausgewählt wird, die Cyclohexan, Cyclooctan, Cyclododecan und Decalin umfasst.

2. Verfahren nach Anspruch 1, **dadurch gekennzeichnet, dass** das zu einer Stufe recycelte Kondensat durch Mischen von Kondensaten oder gasförmigen Phasen erhalten wird, die aus wenigstens zwei Oxidationsstufen stammen.

3. Verfahren nach Anspruch 1 oder 2, **dadurch gekennzeichnet, dass** die am Kopf einer Reaktionsstufe rückgewonnene gasförmige Phase teilweise kondensiert wird und das erhaltene Kondensat zur Speisung der vorhergehenden Stufe recycelt wird.

4. Verfahren nach einem der Ansprüche 1 bis 3, **dadurch gekennzeichnet, dass** die am Kopf der letzten Reaktionsstufe rückgewonnene gasförmige Phase teilweise kondensiert wird und das erhaltene Kondensat zur Speisung der vorhergehenden Stufe recycelt wird.

5. Verfahren nach Anspruch 1, **dadurch gekennzeichnet, dass** der gesättigte Kohlenwasserstoff Cyclohexan ist.

6. Verfahren nach einem der vorhergehenden Ansprüche, **dadurch gekennzeichnet, dass** nach der teilweisen Kondensation der gasförmigen Phasen, welche die Atmosphäre jeder Stufe bilden, die nicht kondensierte Fraktion vollständig kondensiert wird und anschließend das erhaltene Kondensat geflasht wird, um eine wässrige flüssige Fraktion und eine organische Fraktion zu erhalten, wobei die organische Fraktion zu der ersten Oxidationsstufe recycelt wird.

7. Verfahren nach einem der vorhergehenden Ansprüche, **dadurch gekennzeichnet, dass** die Oxidation in einer Anlage durchgeführt wird, die mehrere in Reihe oder kaskadenartig montierte Oxidationsreaktoren umfasst, wobei jeder Reaktor eine Oxidationsstufe bildet.

8. Verfahren nach Anspruch 7, **dadurch gekennzeichnet, dass** die Reaktoren vom Typ Blasensäule sind.

9. Verfahren nach einem der Ansprüche 1 bis 6, **dadurch gekennzeichnet, dass** die Oxidation in einer Anlage durchgeführt wird, die als Oxidationsreaktor eine unterteilte Blasensäule umfasst, wobei jede Unterteilung eine Oxidationsstufe bildet.

## Claims

1. Process for producing alkyl hydroperoxide by oxidizing a saturated hydrocarbon in the liquid phase with an oxygen-containing gas, the reaction being carried out in a plurality of successive steps, the hydrocarbon being supplied at least to the first step at a temperature in the range 100°C to 250°C, at least the last step of the reaction being cooled to maintain and control the temperature in said step at a level which is less than or equal to that of the first step, **characterized in that** each step of the reaction comprises a liquid phase and a gaseous phase forming a gaseous overhead in said step, said gaseous phase forming the overhead being extracted and evacuated to be at least partially condensed, and **in that** the condensate recovered from the gaseous phase of one step is recycled to one of said oxidation steps, wherein the concentration of oxidized products in the supplied reaction medium is at least equal to the concentration of oxidized products in the condensate to be recycled; the process being further **characterized in that** the condensate is recycled directly in at least one step wherein the concentration of oxidized products in the reaction medium supplied in said step is at least equal to the concentration of oxidized products contained in the condensate to be recycled and **in that** the saturated hydrocarbon is selected from the group consisting of cyclohexane, cyclooctane, cyclododecane and decalin.

2. Process according to Claim 1, **characterized in that** the condensate recycled to one step is obtained by mixing condensates or gaseous phases deriving from at least two oxidation steps.

3. Process according to Claim 1 or Claim 2, **characterized in that** the gaseous phase recovered at the head of one reaction step is partially condensed and the condensate obtained is recycled to the supply to the preceding step.

4. Process according to one of Claims 1 to 3, **characterized in that** the gaseous phase recovered at the head of the last reaction step is partially condensed and the condensate obtained is recycled to the supply to the preceding step.

5. Process according to Claim 1, **characterized in that** the saturated hydrocarbon is cyclohexane.

6. Process according to one of the preceding claims, **characterized in that** after partial condensation of the gaseous phases forming the overhead of each step, the uncondensed fraction is condensed completely, then said condensate obtained is flashed in order to obtain an aqueous liquid fraction and an organic fraction, said organic fraction being recycled to the first oxidation step.

7. Process according to one of the preceding claims, **characterized in that** the oxidation is carried out in a plant comprising a plurality of oxidation reactors mounted in series or as a cascade, each reactor forming an oxidation step.

8. Process according to Claim 7, **characterized in that** the reactors are of the bubble column type.

9. Process according to one of Claims 1 to 6, **characterized in that** oxidation is carried out in a plant comprising as the oxidation reactor a partitioned bubble column, each compartment forming an oxidation step.
